(19) Europäisches Patentamt
European Patent Office
Office européen des brevets

(11) EP 4 628 522 A1

(12) EUROPEAN PATENT APPLICATION
published in accordance with Art. 153(4) EPC

(43) Date of publication:
**08.10.2025 Bulletin 2025/41**

(21) Application number: **23897640.1**

(22) Date of filing: **22.11.2023**

(51) International Patent Classification (IPC):
***C08J 3/12*** *(2006.01)*

(52) Cooperative Patent Classification (CPC):
**C08J 3/12**

(86) International application number:
**PCT/JP2023/041954**

(87) International publication number:
**WO 2024/116992 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.11.2022 JP 2022191169**

(71) Applicant: **Dow Toray Co., Ltd.**
**Tokyo 140-8617 (JP)**

(72) Inventors:
- **MATSUSHIMA Hidenori**
  **Ichihara-shi Chiba 299-0108 (JP)**
- **SOUDA Tatsuo**
  **Ichihara-shi Chiba 299-0108 (JP)**
- **OZAKI Kouichi**
  **Ichihara-shi Chiba 299-0108 (JP)**
- **OFUCHI Momoko**
  **Ichihara-shi Chiba 299-0108 (JP)**
- **SUTO Michitaka**
  **Ichihara-shi Chiba 299-0108 (JP)**

(74) Representative: **Murgitroyd & Company**
**165-169 Scotland Street**
**Glasgow G5 8PL (GB)**

# (54) SHAPED SOLID PARTICLES COMPRISING THERMOPLASTIC COPOLYMER, PRODUCTION METHOD FOR SAME, AND COSMETIC INCLUDING SAME

(57) An object of the present invention is to provide molded solid particles made of a thermoplastic copolymer having a silicon-containing organic group, which has excellent solubility in solvents acceptable for cosmetic materials and excellent handling properties, and can be mass-produced by an industrial production process, as well as uses and production methods thereof.

SOLUTION

Molded solid particles, comprising a thermoplastic copolymer made by copolymerizing: (a1) an unsaturated monomer having one radical polymerizable organic group and one silicon-containing organic group in the molecule; (a2) an unsaturated monomer having one radical polymerizable vinyl group in the molecule, which is different from component (a1); wherein the major axis of the particles is less than 10 mm, as well as a use thereof, and a production method of the molded solid particles including a stranding step and a cutting step.

[FIG. 1]

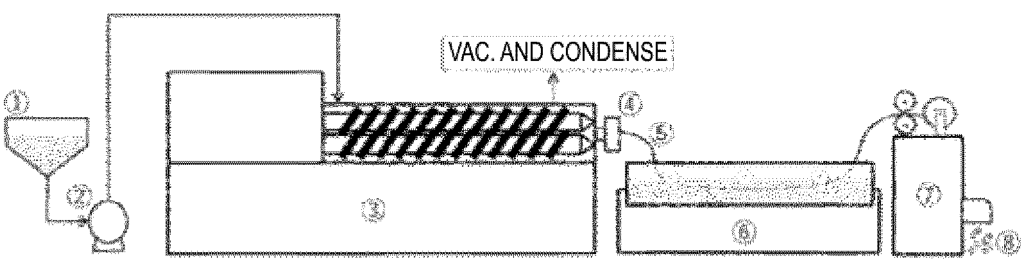


EP 4 628 522 A1

## Description

### TECHNICAL FIELD

The present invention relates to molded solid particles of a thermoplastic copolymer having a silicon-containing organic group in a molecule, which have excellent solubility during use, and a method for producing the same. The present invention also relates to a use of the molded solid particles as a cosmetic raw material or a film forming agent, and to cosmetic materials containing the same.

### BACKGROUND ART

It has been known for some time that copolymers having silicon-containing organic groups, such as a carbosiloxane dendrimer structure, which have excellent film forming properties, are used in cosmetic materials for the purpose of improving the makeup staying power and improving the water resistance, sebum resistance, and the like of cosmetic materials (for example, Patent Document 1 and the like). These copolymers having silicon-containing organic groups are formed by radical copolymerization of unsaturated monomers having silicon-containing organic groups, such as carbosiloxane dendrimer structures, with other unsaturated monomers.

These copolymers having silicon-containing organic groups are generally used as cosmetic raw materials in the form of a solution or dispersion by dissolving or uniformly dispersing in a soluble silicone-based solvent (such as a volatile linear or cyclic dimethylpolysiloxane) or other organic solvent (such as isododecane) (see Patent Document 1 and the like). However, while the solvent, which is a carrier, improves the handling and workability of the copolymer, the solvent may limit the freedom and diversity of formulation design in relation to cosmetic formulations and other additives. For example, organic solvents such as isododecane act as plasticizers and may degrade the feel when used in cosmetic materials containing the copolymer. In addition, solvents that are acceptable as carriers for cosmetic raw materials generally tend to have low flash points, requiring careful safety management during transportation and handling.

On the other hand, the use of silicone acrylate copolymers in a solid form that does not contain a solvent as a carrier has been proposed. For example, Patent Document 2 proposes removing the solvent from a commercially available silicone acrylate copolymer solution, crushing the resulting solid by hand, and then dissolving the solid in a plant-derived oil agent for cosmetic materials. However, this method is not suitable for industrial production processes, and since the solubility of the solid ground material in various oil agents is insufficient, even small amounts require long-term shearing operations under heated conditions, making the material extremely difficult to handle and work with as a cosmetic raw material.

Furthermore, Patent Document 1 also proposes pulverizing the solid silicone acrylate copolymer obtained by removing the solvent in a similar manner using a ball mill. However, the silicone acrylate copolymer in Patent Document 1 has a low glass transition temperature and is soft, and therefore tends to aggregate during a grinding operation that involves frictional heat (see, for example, Comparative Example 3 of the present application described below), and is extremely poor in terms of handling and workability as a cosmetic raw material.

In response, Patent Document 3 proposes a method of preparing a silicone methacrylate emulsion which is then dried with a spray dryer to obtain silicone methacrylate particles. Here, a crosslinkable monomer having two or more (meth)acrylic groups in the molecule is used as the raw material for silicone methacrylate, and the resulting silicone methacrylate particles do not have thermoplasticity. As a result, particle aggregation as described in Patent Document 1 is suppressed, but the resulting silicone methacrylate particles are not soluble in solvents and cannot be uniformly dispersed in a formulation as a cosmetic raw material.

As described above, none of the aforementioned documents discloses molded particles of a copolymer having a silicon-containing organic group that has good solubility in a solvent, can be uniformly dispersed in a formulation as a cosmetic raw material, can be mass-produced using an industrial production process, and has excellent handling and workability as a cosmetic raw material.

### RELATED ART DOCUMENTS

#### Patent Documents

Patent Document 1: Japanese Unexamined Patent Application 2000-63225
Patent Document 2: Japanese Unexamined Patent Application 2018-518505
Patent Document 3: Japanese Unexamined Patent Application 2009-149880

## SUMMARY OF THE INVENTION

### PROBLEM TO BE SOLVED BY THE INVENTION

In view of the above problems and the current technical situation, an object of the present invention is to provide molded solid particles made of a thermoplastic copolymer having a silicon-containing organic group, which has excellent solubility in solvents acceptable for cosmetic materials and excellent handling properties, and which can be mass-produced by an industrial production process. Another object of the present inventionis to provide a use for the molded solid particles, and a production method thereof.

### MEANS FOR SOLVING THE PROBLEM

As a result of intensive investigations, the present invention inventors have found that the aforementioned problems can be solved by molded solid particles, containing a thermoplastic copolymer made by copolymerizing:

(a1) an unsaturated monomer having one radical polymerizable organic group and one silicon-containing organic group in the molecule; and
(a2) an unsaturated monomer having one radical polymerizable vinyl group in the molecule, which is different from component (a1) (in other words, a copolymer having a silicon-containing organic group and no crosslinking sites in the molecule);
wherein the major axis of the particles is less than 10 mm. Thus the present invention was achieved. The present inventors also found that the aforementioned problems can be solved by using the molded solid particles as a cosmetic raw material, a cosmetic material containing the molded solid particles, and a method for producing a cosmetic material including a step of dissolving or dispersing the molded solid particles in a solvent acceptable for cosmetic materials, and thus arrived at the present invention.

Similarly, the present invention inventors have found that the aforementioned problems can be solved by a method of producing molded solid particles according to the present invention, which includes a step (I) of extruding the aforementioned thermoplastic copolymer from a die in the form of strands, and a step (II) of cutting the strand-like extrusion product obtained in step (I), and thus achieved the present invention.

### EFFECT OF THE INVENTION

The present invention can provide molded solid particles made of a thermoplastic copolymer having a silicon-containing organic group, which has excellent solubility in solvents acceptable for cosmetic preparations, excellent handling properties, and can be mass-produced by an industrial production process.

In particular, the thermoplastic copolymer having a silicon-containing organic group according to the present invention has excellent solubility due to not having crosslinked moieties within the molecule, and is free of solvents that serve as carriers, so when used as a cosmetic raw material, the copolymer has the advantage of not restricting the freedom and diversity of cosmetic formulation design. Furthermore, the molded solid particles according to the present invention is solvent-free, so there is no risk of ignition due to flammable solvents. Furthermore, by designing a copolymer with an appropriate glass transition point (Tg), the copolymer will have excellent processability, such as cutting and the like, and can avoid the problem of aggregation. This has the advantage that a cosmetic raw material or a film forming agent (not limited to cosmetic applications) can be provided that not only has good solubility but also further improves handling workability, processability, formulation stability, and the like.

Similarly, the method for producing molded solid particles according to the present invention is excellent for mass productivity and can be applied to a continuous production process by using an extruder (including a single-screw or multi-screw extruder) equipped with a die and appropriately controlling the temperature during extrusion and the temperature inside the barrel, and can provide a production method that avoids aggregation and processability problems such as molding and cutting, and further improves quality and production stability.

### BRIEF DESCRIPTION OF THE DRAWINGS

FIG. 1 is an overall view of a production device used for producing the molded solid particles of the present invention.

## DESCRIPTION OF THE PREFERRED EMBODIMENTS

**In** the present specification, "(meth)acrylic acid" indicates that both acrylic acid and methacrylic acid are included. Similarly, "(meth)acrylate", "(meth)acryloxy", and "(meth)acrylamide" also indicate that both acrylate and methacrylate, acryloxy and methacryloxy, and acrylamide and methacrylamide, respectively, are included. **In** the present specification, "cosmetic material" and "cosmetic product" are used interchangeably.

[Thermoplastic copolymer]

**The** molded solid particles of the present invention contain a thermoplastic copolymer obtained by copolymerizing component (a1): an unsaturated monomer having one radical polymerizable organic group and one silicon-containing organic group in the molecule, and component (a2): an unsaturated monomer different from component (a1) having one radical polymerizable vinyl group in the molecule. Herein, the components (a1) and (a2) are both non-crosslinkable monomers, and the resulting copolymer does not have a crosslinked structure in the molecule and has thermoplasticity. Therefore, the molded solid particles of the present invention has good solubility (hereinafter, sometimes referred to as being "easily soluble") in solvents that are acceptable for cosmetic preparations.

The silicon-containing organic group in component (a1) is a functional group that imparts water repellency to the entire copolymer and, when the molded solid particles are used as a cosmetic raw material (particularly a film forming agent), improves the cosmetic staying power of the cosmetic material, and improves the water resistance, sebum resistance, and the like. The silicon-containing organic group is not particularly limited, but is preferably has at least one structure selected from a carbosiloxane dendrimer structure and a siloxane macromonomer structure having a chain polysiloxane structure.

An example of the siloxane macromonomer structure is a chain organosiloxane group expressed by the following formula:

[Chem. Fig. 1]

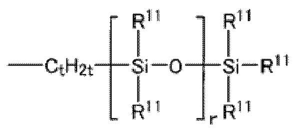

[Chem. Fig. 2]

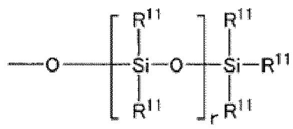

(where $R^{11}$ each independently represents a substituted or unsubstituted monovalent hydrocarbon group having 1 to 30 carbon atoms, a hydroxyl group, or a hydrogen atom, but at least one of the $R^{11}$ groups is a monovalent hydrocarbon group. t is a number in a range of 2 to 10, and r is a number in a range of 1 to 500).

An example of the carbosiloxane dendrimer structure is a silylalkyl group having a siloxane dendron structure expressed by the following formula (1).

[Chem. Fig. 3]

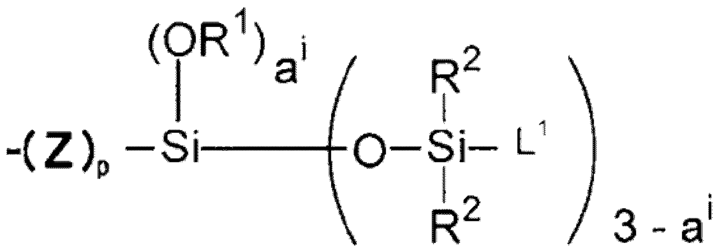

(1)

{where

Z represents a divalent organic group;
P represents 0 or 1;
$R^1$ and $R^2$ independently represent an alkyl group having 1 to 10 carbon atoms, an aryl group, or an aralkyl group; and
$L^1$ represents a silyl alkyl group as expressed by the following Formula (2) when i = 1

[Chem. Fig. 4]

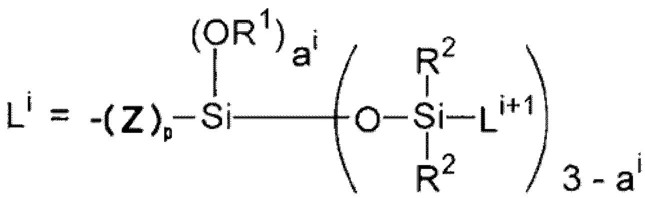

(2)

(where
Z and p are the same as described above;
$R^1$ and $R^2$ are the same as described above;
i represents an integer from 1 to 10 indicating the total hierarchical number of silyl alkyl groups; and
$L^{i+1}$ represents a group selected from a group consisting of a hydrogen atom, alkyl groups having 1 to 10 carbon atoms, aryl groups, aralkyl groups, and the silylalkyl groups; however, when i = c (c represents an integer from 1 to 10 indicating the hierarchy of the silylalkyl group), $L^{i+1}$ represents a hydrogen atom, an alkyl group having 1 to 10 carbon atoms, an aryl group, or an aralkyl group, but represents the silylalkyl group when i < c, and $a^i$ represents an integer from 0 to 3)}.

Note that the carbosiloxane dendrimer structure is a chemical structure that is highly branched radially from a single silicon atom, and i, which indicates the total number of silyl alkyl groups in the hierarchy, indicates the degree of branching. For example, if the total number of hierarchies i is 1 and $L^{i+1}$ is, for example, a methyl group, then the carbosiloxane dendrimer structure refers to the following structure.

[Chem. Fig. 5]

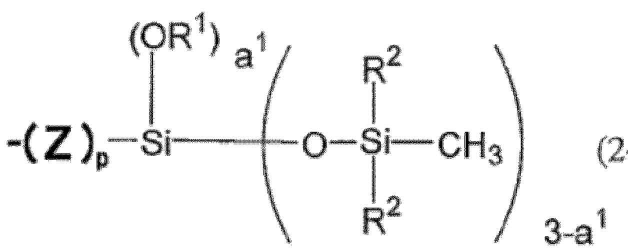

(2-1)

(where Z, p, $R^1$, and $R^2$ are the same as described above, and $a^1$ represents an integer between 0 and 3)

Similarly, if the hierarchy i is 2 and $L^{i+1}$ is, for example, a methyl group, then the carbosiloxane dendrimer structure refers to the following structure (however, p = 1).

[Chem. Fig. 6]

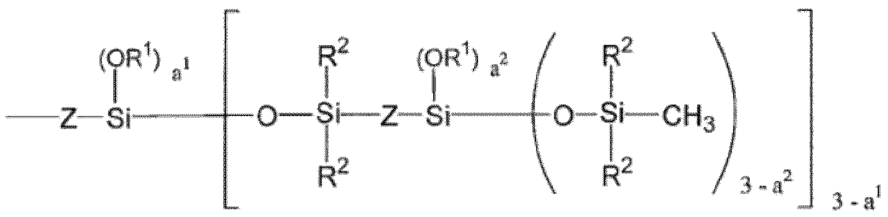

(2-2)

(where Z, $R^1$, and $R^2$ are the same as described above, and $a^1$ and $a^2$ represent an integer between 0 and 3)

Preferably, the aforementioned a, $a^1$, and $a^2$ are 0, and the carbosiloxane dendrimer structure is particularly preferably as follows.

[Chem. Fig. 7]

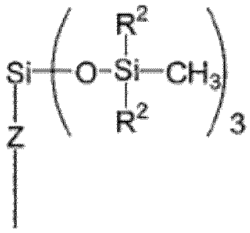

(Where Z and $R^2$ are the same as described above)

[Chem. Fig. 8]

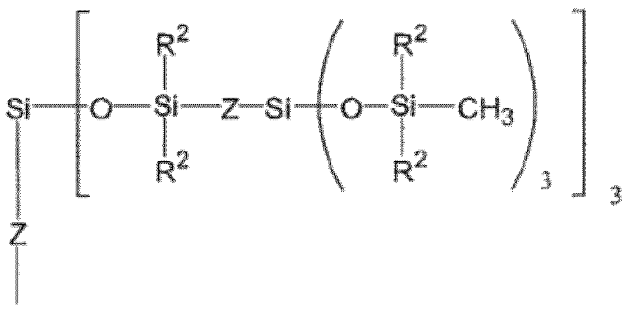

(Where Z and $R^2$ are the same as described above)

The unsaturated group of component (a1) is not limited so long as one radical polymerizable unsaturated bond is provided in the molecule, and examples include vinyl groups, allyl groups, (meth)acrylic groups, and the like. In particular, component (a1) is preferably an acrylic acid ester-based monomer or a methacrylic acid ester-based monomer having a silicon-containing group as described above. Of these, a particularly preferable unsaturated monomer is an unsaturated monomer where an unsaturated group as expressed by the following structures is bonded to a silicon atom (Si) directly or through - (Z) p- or -Z- in the carbosiloxane dendrimer structure described above.

An organic group containing an acrylic group or methacrylic group as expressed by

[Formula 9]

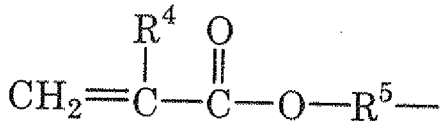

(Where $R^4$ represents a hydrogen atom or a methyl group, and $R^5$ represents an alkylene group having 1 to 10 carbon atoms.),
or

[Formula 10]

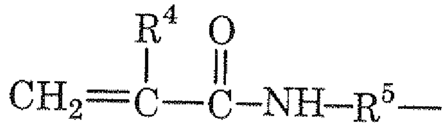

(Where $R^4$ and $R^5$ are the same as described above.).

More specifically, examples of this component include unsaturated monomers containing a silicon-containing organic group expressed by the average composition formulas shown below. These components can be used alone or in combination of two or more types as component (a1), and the resulting copolymer contains a silicon-containing organic group derived from component (a1).

[Chem. Fig. 11]

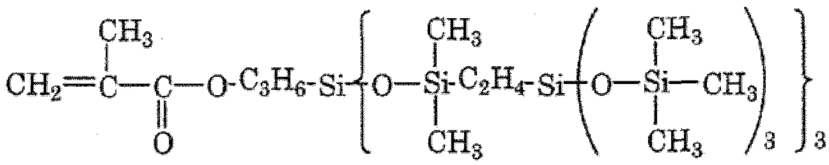

(In other words,

[Chem. Fig. 12]

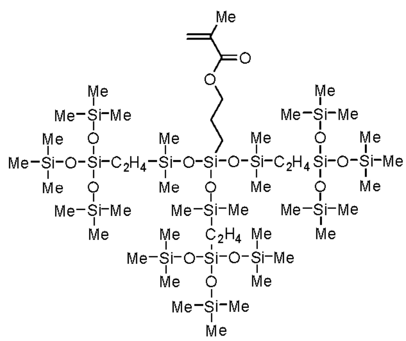

(where Me represents a methyl group))

[Chem. Fig. 13]

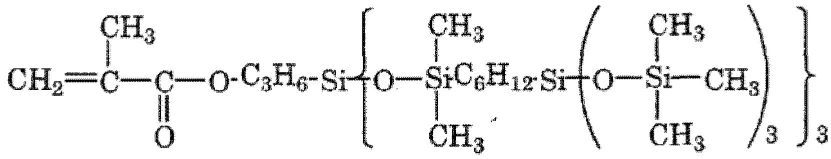

[Chem. Fig. 14]

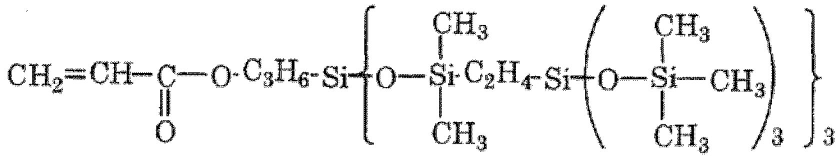

[Chem. Fig. 15]

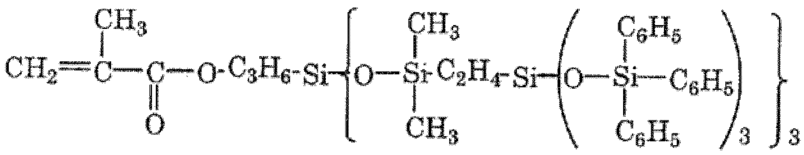

[Chem. Fig. 16]

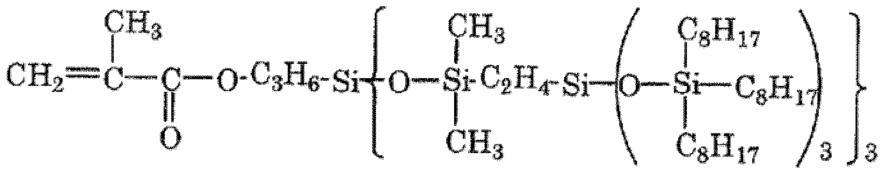

[Chem. Fig. 17]

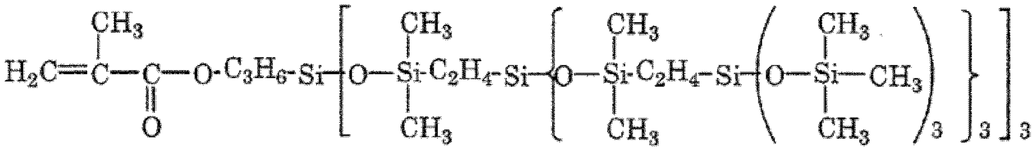

[Chem. Fig. 18]

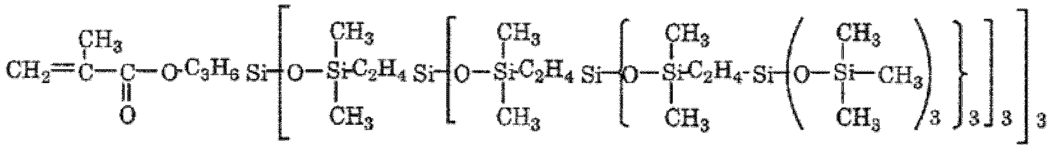

[Chem. Fig. 19]

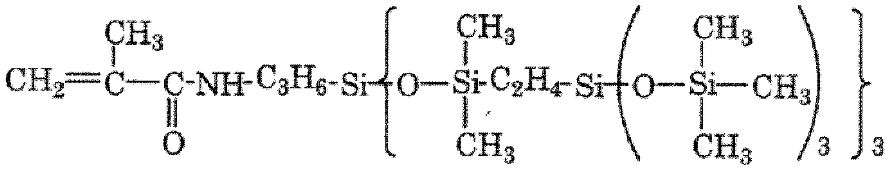

[Chem. Fig. 20]

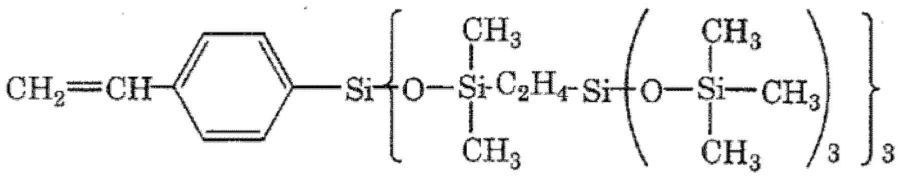

[Chem. Fig. 21]

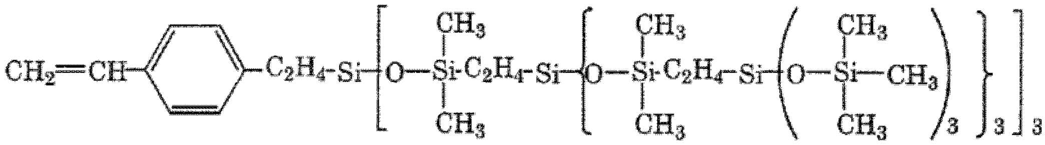

[Chem. Fig. 22]

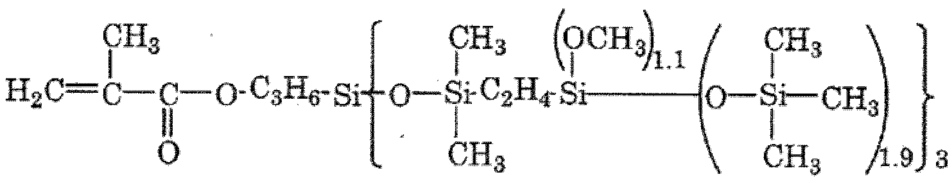

[Chem. Fig. 23]

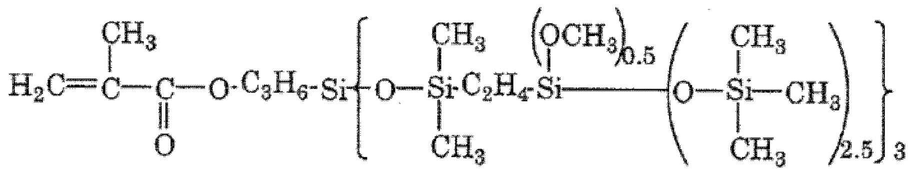

[Chem. Fig. 24]

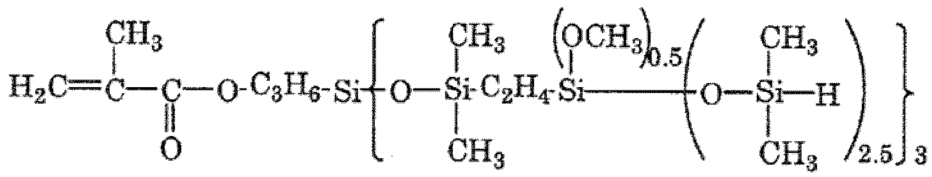

[Chem. Fig. 25]

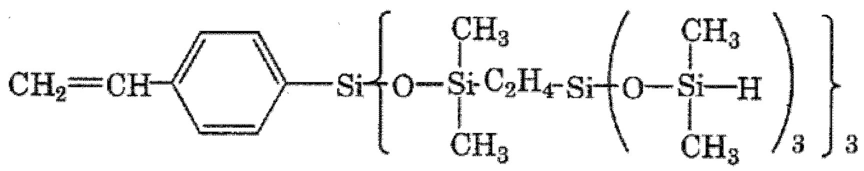

[Chem. Fig. 26]

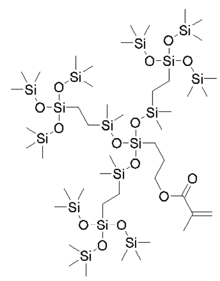

(where, "Si-" at the molecular terminus refers to "Si-$CH_3$")

[Chem. Fig. 27]

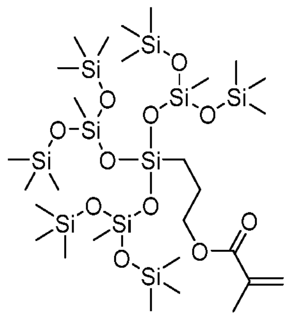

(where, "Si-" at the molecular terminus refers to "Si-$CH_3$")

[Chem. Fig. 28]

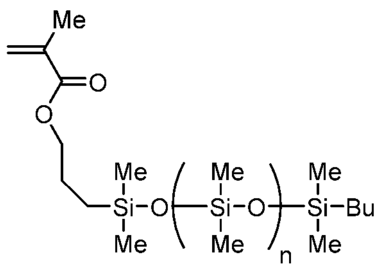

(where Me is a methyl group, n is a positive number, and Bu is a butyl group)

[Chem. Fig. 29]

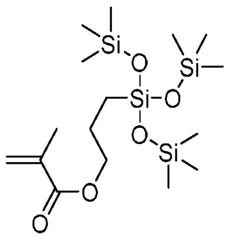

(where, "Si-" at the molecular terminus refers to "Si-$CH_3$")

Component (a2) constituting the thermoplastic copolymer of the present invention is an unsaturated monomer different from component (a1) and has one radical polymerizable vinyl group in the molecule, and there are no particular limitations on the type, or the like. More specifically, component (a2) is a vinyl monomer having one vinyl group in the molecule, but not having a silicon-containing organic group such as a carbosiloxane dendrimer structure or the like in the molecule.

Examples of the vinyl-based monomer include monomers that are the raw materials of organic resins, which are generally called vinyl-based resins. Specific examples include: lower alkyl (meth)acrylates such as methyl (meth)acrylate, ethyl (meth)acrylate, n-propyl (meth)acrylate, and isopropyl (meth)acrylate; glycidyl (meth)acrylate; higher (meth)acrylates such as n-butyl (meth)acrylate, isobutyl (meth)acrylate, tert-butyl (meth)acrylate, n-hexyl (meth)acrylate, cyclohexyl (meth)acrylate, 2-ethylhexyl (meth)acrylate, octyl (meth)acrylate, lauryl (meth)acrylate, and stearyl (meth)acrylate; lower fatty acid vinyl esters such as vinyl acetate and vinyl propionate; higher fatty acid esters such as vinyl butyrate, vinyl caproate, vinyl 2-ethylhexanoate, vinyl laurate, and vinyl stearate; aromatic vinyl-based monomers such styrene, vinyl toluene, benzyl (meth)acrylate, phenoxyethyl (meth)acrylate, and vinyl pyrrolidone; vinyl-based monomers containing an amide group such as (meth)acrylamide, N-methylol (meth)acrylamide, N-methoxymethyl (meth)acrylamide, isobutoxymethoxy (meth)acrylamide, and N,N-dimethyl (meth)acrylamide; vinyl-type monomers containing a hydroxy group such as 2-hydroxyethyl (meth)acrylate, 2-hydroxybutyl (meth)acrylate, and 2-hydroxypropyl (meth)acrylate: fluorine-containing vinyl-type monomers such as trifluoropropyl (meth)acrylate, perfluorobutylethyl (meth)acrylate, and perfluorooctylethyl (meth)acrylate; vinyl-type monomers containing an epoxy group such as glycidyl (meth)acrylate and 3,4 epoxycyclohexylmethyl (meth)acrylate; carboxylic acid-containing vinyl-type monomers such as (meth)acrylic acid, itaconic acid, crotonic acid, fumaric acid, and maleic acid; ether bond-containing vinyl-based monomers such as tetrahydrofurfuryl (meth)acrylate, butoxyethyl (meth)acrylate, ethoxydiethylene glycol (meth)acrylate, polyethylene glycol (meth)acrylate, polypropylene glycol mono(meth)acrylate, hydroxybutyl vinyl ether, cetyl vinyl ether, and 2-ethylhexyl vinyl ether; silicone compounds containing an unsaturated group such as (meth)acryloxypropyl trimethoxysilane, (branched or linear) polydimethyl siloxane containing a (meth)acrylic group at one end, and a polydimethyl siloxane containing a styryl group at one end; butadiene; vinyl chloride; vinylidene chloride; (meth)acrylonitrile; dibutyl fumarate; maleic anhydride; dodecyl succinic anhydride; (meth)acrylic glycidyl ether; alkali metal salts, ammonium salts, organic amine salts of radical polymerizable unsaturated carboxylic acids such as (meth)acrylic acid, itaconic acid, crotonic acid, fumaric acid, and maleic acid; radical polymerizable unsaturated monomers having sulfonic acid groups such as styrene sulfonic acid, and their alkali metal salts, ammonium salts, organic amine salts; and quaternary ammonium salts derived from (meth)acrylic acids, such as 2-hydroxy-3-methacryloxypropyl trimethylammonium chloride, methacrylates of alcohols with tertiary amine groups, such as diethylamine methacrylates, and quaternary ammonium salts thereof.

Furthermore, an organic silicon compound having a vinyl-based radical polymerizable unsaturated group and a hydrolyzable group can also be used. **In** this case, the film strength is stronger and the water repellency durability is improved, which is preferable.

Similarly, unsaturated monomers having at least one acidic group in the molecule, or a salt thereof, can also be used. The unsaturated monomer having at least one acidic group in each molecule, or a salt thereof, is a compound having a radical polymerizable vinyl group and at least one acidic group in each molecule, or a salt thereof. Examples of the acidic group include groups from carboxylic acid, sulfonic acid, phosphonic acid, and the like. Examples of salts thereof include alkali metal salts, alkaline earth metal salts, basic amino acid salts, ammonium salts, alkyl ammonium salts, alkyl amine salts, alkanolamine salts, and the like. Specific examples include sodium salt, potassium salt, magnesium salt, calcium salt, L-arginine salt, L-histidine salt, L-lysine salt, ammonium salt, triethanolamine salt, aminomethyl propanediol salt, and complex salts thereof. Compounds that have these acidic groups release protons (H+) in aqueous solutions at specific pH values, or combine with cationic components in the solution to form salts, thereby changing the hydrophilicity or hydrophobicity of the compound. Compounds having salts of acidic groups similarly undergo dissociation of the salt at a particular pH and exhibit a change in the hydrophilicity or hydrophobicity of the compound. Therefore, the cosmetic raw material can easily be washed off during washing by adding an appropriate amount of these compounds having an acidic group or a salt thereof to the cosmetic raw material, thereby further improving the effect of exhibiting good cosmetic retention.

Similarly, an unsaturated monomer containing a fluorine-containing organic group such as a perfluoroalkyl group can also be added for the purpose of improving the water repellency of the thermoplastic copolymer containing a silicon-containing organic group of the present invention. An example is a vinyl-based monomer such as an acrylic monomer, a methacrylic monomer, or the like having an organic group containing fluorine such as a perfluoroalkyl group or the like.

The copolymer of the present invention is obtained by copolymerizing of the aforementioned component (a1) and component (a2), and the mass ratio at the time of the copolymerization is preferably within a range of (a1) : (a2) = 10:90 to 90:10, more preferably 20:80 to 85:15, and even more preferably 30:70 to 60:40. In particular, the mass% of the component (a1) described above is at least 20 mass%, and preferably at least 30 mass% relative to the total mass of component (a1) and component (a2), and component (a1) is particularly preferably 20 mass% to 60 mass% of total monomer units.

The thermoplastic copolymer containing a silicon-containing organic group according to the present invention can be produced by a method that includes a step of adding a polymerization initiator to a raw material composition containing the monomer to carry out a polymerization reaction. Thereafter, a step may be provided in which the optionally obtained polymerization reaction product is contacted with a nickel catalyst or a palladium catalyst to perform a hydrogenation reaction.

The polymerization method used in the polymerization reaction may be radical polymerization, anionic polymerization, cationic polymerization, group transfer polymerization, organometallic mediated radical polymerization, or atom transfer radical addition, but radical polymerization is preferred. The radical polymerization method can be carried out via at least one liquid phase polymerization reaction selected from solution polymerization, suspension polymerization, mini-emulsion polymerization, and emulsion polymerization, but the solution polymerization method is preferably used. The solution polymerization is performed by reacting the monomer composition containing component (a1) and component (a2) in a solvent at a temperature of 50 to 150°C for 3 to 20 hours in the presence of a radical initiator. Examples of the solvent used in the polymerization reaction include aliphatic hydrocarbons such as hexane, octane, decane, cyclohexane, methylcyclohexane, isoparaffin, and the like; aromatic hydrocarbons such as benzene, toluene, xylene, and the like; ethers such as diethyl ether, diisopropyl ether, dibutyl ether, tetrahydrofuran, dioxane, and the like; ketones such as acetone, methyl ethyl ketone, methyl isobutyl ketone, diisobutyl ketone, and the like; esters such as methyl acetate, ethyl acetate, butyl acetate, isobutyl acetate, and the like; alcohols such as methanol, ethanol, isopropyl alcohol, butanol,

and the like; and organosiloxane oligomers such as octamethylcyclotetrasiloxane, decamethylcyclopentasiloxane, hexamethyldisiloxane, octamethyltrisiloxane, and the like. One of these solvents may be used alone, or two or more types thereof may be used in a mixture. Note that as described later, the organic solvent used in the polymerization reaction is finally removed in the process of forming the molded solid particles of the present invention.

The radical initiator can be a conventionally known compound generally used in a radical polymerization method. Specific examples thereof include azobis compounds such as 2,2'-azobis(isobutyronitrile), 2,2'-azobis(2-methylbutyronitrile), and 2,2'-azobis(2,4-dimethylvaleronitrile); and organic peroxides such as benzoyl peroxide, lauroyl peroxide, tert-butyl peroxybenzoate, tert-butylperoxy-2-ethylhexanoate, tert-hexylperoxy-2-ethylhexanoate, tert-amylperoxypivalate, tert-butylperoxypivalate, t-hexylperoxypivalate, di(4-t-butylcyclohexyl)peroxydicarbonate, di(3,5,5-trimethylhexanoyl)peroxide, diisopropylperoxydicarbonate, and the like. One type of these radical initiators may be used alone, or two or more types may be mixed and used together. **The** usage amount of the radical initiator is preferably in the range of from 0.1 to 10 parts by weight per a total of 100 parts by weight of the monomer composition.

**In** addition, a chain transfer agent can be added during polymerization. Specific examples of the chain transfer agent include: mercapto compounds such as 2-mercaptoethanol, butyl mercaptan, n-dodecyl mercaptan, 3-mercaptopropyltrimethoxysilane, polydimethylsiloxane having a mercaptopropyl group, and mercaptopropionic acid; halides such as methylene chloride, chloroform, carbon tetrachloride, butyl bromide, and 3-chloropropyltrimethoxysilane; secondary alcohols such as isopropyl alcohol and glycerin; sulfurous acid (salts) such as sodium sulfite; and sulfurous acid (salts). Examples of the sulfite (salt) such as sodium hydrogen sulfite; sodium dithionite; potassium pyrosulfite; hydrogen peroxide; dithionates (salts) such as sodium dithionite; pyrosulfite (salts) such as potassium pyrosulfite; and hydrogen peroxide.

The polymerization reaction product can be contacted with a nickel catalyst or palladium catalyst, which are hydrogenation reaction catalysts. By being contacted with these catalysts, the vinyl groups of unreacted monomers remaining in the polymerization reaction product are saturated, and irritation and odor when added to the cosmetic material can be reduced. Nickel catalysts include, but are not necessarily limited to, nickel/diatomaceous earth and raney nickel catalysts. Examples of palladium catalysts include palladium compounds such as tetrakis (triphenylphosphine)palladium (0) and dichlorobis (triphenylphosphine)palladium (II); carbon-supported palladium, carbon-supported palladium hydroxide, and platinum oxide, but the palladium catalyst is not necessarily limited to these.

The temperature when contacting the polymerization reaction product with the nickel catalyst or palladium catalyst is 50 to 200 °C, and preferably 70 to 130 °C. The pressure is from 1 to 1000 kg/cm$^2$ (absolute pressure), and preferably from 2 to 100 kg/cm$^2$. The contact time is from 1 to 15 hours, and preferably from 3 to 10 hours. The reaction can be performed in a solvent, and the solvent that is used during polymerization may be used as is, or the solvent may be substituted. The solvents that can be used are the same as those described for the polymerization reaction.

[Copolymer properties]

The copolymer described above does not have a crosslinked structure in the molecule, and therefore has thermoplastic properties and is easily soluble in solvents acceptable for cosmetic preparations. In the present invention, a copolymer that does not have thermoplasticity and has a crosslinked structure in the molecule is generally insoluble or poorly soluble in solvents, and therefore cannot achieve the technical effects of the present invention. The molded solid particles containing the copolymer of the present invention are preferably obtained by a continuous production method including a step of extruding the copolymer while molding after melting, as described below. For example, the copolymer can be molded by extruding in the form of strands or by extruding using a droplet former. However, the manufacturing method of the molded solid particles, particularly the molding means, is not particularly limited, and naturally, optimal manufacturing conditions can be set according to the intended molding means and manufacturing equipment. Furthermore, the molding means includes cutting or pulverizing the extruded copolymer, and the molding process may be carried out at any timing before, during, or after cooling.

The copolymer according to the present invention has a glass transition point (Tg) of the copolymer itself excluding the solvent, which is preferably in a range of 30 to 120°C, more preferably in a range of 35 to 100°C, and particularly preferably in a range of 40 to 90°C. If the Tg of the copolymer exceeds the upper limit, the solubility of the obtained molded solid particles in a solvent may decrease, and, for example, the hardness of the solid copolymer extruded in the form of a strand may increase, so reheating the strand will be required after cooling and cutting, thereby decreasing industrial productivity. On the other hand, if the Tg of the copolymer is less than the above lower limit, particularly if the copolymer is applied to a process for producing molded solid particles having a step of extruding the copolymer in the form of strands as described below, the melt viscosity of the copolymer will drop suddenly, and molding defects or deformation may occur when the copolymer is molded into strands. Furthermore, if the Tg of the copolymer is low, the strand-like molded product extruded from the die may be too soft and prone to poor cutting, so adjusting the cooling state to a temperature suitable for cutting must be performed before cutting, which may result in reduced industrial productivity. Furthermore, if the Tg of the copolymer is less than the above lower limit, the amount of remaining solvent tends to be large

in the step of distilling off (removing) the solvent, and the solvent may remain in the obtained molded solid particles, which may impair the suitability, performance, and usability as a cosmetic raw material, as well as cause quality defects such as surface tack, aggregation, and deformation of the molded solid particles.

From the viewpoint of moldability, the copolymer according to the present invention has a melt viscosity in a range of 0.1 to 10,000 Pa·s at the temperature during extrusion (for example, a temperature range of 35 to 220°C, which is 5 to 100°C higher than the Tg).

The melt viscosity range during the step of extruding the resin in the form of strands from a die is preferably 1 to 10000 Pa·s, more preferably 2.5 to 8500 Pa·s, and particularly preferably 5 to 7000 Pa·s. If the temperature at the die outlet is too high, the strand-shaped molded product is likely to suffer molding defects or deformation.

The melt viscosity range in the step of ejecting droplets with a droplet forming device is preferably 0.1 to 100 Pa·s, more preferably 0.5 to 80 Pa·s, and particularly preferably 1 to 60 Pa·s. If the melting temperature of the droplet forming device is too low, droplets may not be ejected, which may result in molding defects or deformation.

[Optional Fillers and Additives]

The copolymer of the present invention may be blended with any filler or additive depending on the application and required performance as a cosmetic raw material and within a range that does not impair the technical effects of the present invention, and may form molded solid particles containing these fillers and additives. Specifically, one or more fillers selected from glass fiber, carbon fiber, glass cloth, calcium carbonate, mica, talc, and the like may be added. Furthermore, one or more additives selected from strength improvers, antioxidants, ultraviolet light absorbers, light resistance stabilizers, heat resistance stabilizers, plasticizers, foaming agents, crystal nucleating agents, lubricants, antistatic agents, conductivity imparting agents, colorants (including pigments, dyes, and the like), compatibilizers, flame retardants, antifungal agents, low-shrinkage agents, thickeners, release agents, anti-fogging agents, bluing agents (coloring agents for the purpose of transparency), silane coupling agents, and the like may be added. These components are preferably uniformly mixed or kneaded with the copolymer of the present invention, and then formed into molded solid particles. These components may be added to a solution containing the copolymer of the present invention, or may be added to molten copolymer after removing the solvent or while removing the solvent, and then kneaded. Note that although the aforementioned fillers are insoluble in solvents acceptable for cosmetic materials, when blended in the molded solid particles of the present invention in a state of being uniformly dispersed in the copolymer of the present invention, there is an advantage in that when the thermoplastic copolymer constituting the molded solid particles dissolves in the solvent, the insoluble fillers supported by the copolymer are uniformly dispersed in the cosmetic formulation to form a preparation.

[Molded solid particles and manufacturing method thereof]

The molded solid particles of the present invention are made of the aforementioned thermoplastic copolymer and optional additives, and have a major axis of less than 10 mm. The shape of the molded solid particles is not particularly limited as long as the particles are molded into a certain shape, but one or more shapes selected from pellets, tablets, short fibers, and molded grains are preferred, but from the viewpoint of the production method described below, pellets and short fibers are particularly preferred. The major axis of a particle refers to the longest diameter of the particle, and when the particle is molded into, for example, a cube, a rectangular parallelepiped, a cylinder, a sphere, an elongated sphere, a hemisphere, or an irregular shaped pulverized product, the major axis can be any diameter, including the actual diameter or a length. When a large number of molded solid particles are included, the average value of the major axis may be used, as determined by observation under an electron microscope, or the settings of a pelletizer (cutting device).

However, from the viewpoint of the solubility of the molded solid particles, the molded solid particles of the present invention must have a major axis (average major axis when a large number of particles are present) of less than 10 mm, and from the viewpoint of industrial productivity, the major axis may be in a range of 0.1 to 9.5 mm, 0.5 to 9.0 mm, or 0.5 to 7.5 mm.

The molded solid particles according to the present invention preferably have a non-volatile component content of 97 mass% or more, and particularly preferably 98 to 100 mass% of non-volatile components. Non-volatile components include any fillers and additives that are non-volatile. On the other hand, if a large amount of volatile components such as the organic solvent or low molecular weight monomers used during synthesis remain in the molded solid particles, the obtained molded solid particles (e.g., cut pellets obtained by the method described below) are likely to be tacky on the surface and are likely to form non-uniform lumps due to aggregation or deformation of the molded solid particles, which may cause poor appearance and quality, degraded handling workability, and reduced solubility in solvents that are acceptable for cosmetic materials. Furthermore, if the amount of remaining solvent is high, volatile contaminants derived from the remaining solvent may cause fire or impair the feel and quality of the resulting cosmetic material.

The method for producing the molded solid particles of the present invention is not particularly limited, but from the viewpoint of quality and industrial productivity, a preferable production method includes a step (I) of extruding a

thermoplastic copolymer from a die in the form of strands, and a step (II) of cutting the strand-like extrusion product obtained in step (I). Similarly, another method for producing the molded solid particles of the present invention may include a process including a step (III) of extruding a thermoplastic copolymer from a droplet former, and a step (IV) of cooling and solidifying the extrusion product obtained in step (III). The production method will now be described in detail. Incidentally, the present production method can be carried out either batchwise or continuously, but from the standpoint of productivity and quality control, a continuous method is preferable using an extruder equipped with a die and a cutting device, which will be described later.

Step (I) is a step in which the thermoplastic copolymer of the present invention is melted and extruded from holes in a die to form a strand-like extrusion product. The strand-like extrusion product is obtained as a noodle-like or thin rod-like solid or semi-solid copolymer having thermoplasticity, and because the material is hot when extruded, the material is preferably cooled using a cooling bath such as a water bath or air cooling (including cooling transport such as a belt conveyor or the like). The temperature of the cooled strands is preferably adjusted to achieve a hardness that allows the strands to be easily cut in a subsequent step (II) of cutting the strand-like extrusion product, but forming strands is not necessary, and in this case, the material will be cut in the subsequent step (II) of cutting the extrusion product.

The thickness of the strand-like extrusion product is determined by the diameter of the die holes. From the viewpoint of the ease of solubility of the molded solid particles obtained by this production method, the die hole diameter must be less than 10 mm. In order to facilitate cutting of the strand-like extruded product in the subsequent step (II) of cutting the strand-like extrusion product, the die hole diameter is preferably in a range of 1.0 to 7.5 mm, and particularly preferably in a range of 3 to 7 mm. The number of strand-like extrusion products extruded from the die is determined by the number of holes in the die, and is not particularly limited. However, from the viewpoint of production efficiency, the number of holes in the die is preferably in a range of 2 to 50, 2 to 20, or 2 to 10. Even when strands are not formed, a die hole size equivalent to the above conditions can be used. The shape of the holes in the die may be any shape, such as a circle or a polygon (including an asymmetric hole shape), for the purpose of increasing cooling efficiency and improving convenience during use.

In step (I), the thermoplastic copolymer of the present invention is extruded from the holes of the die in a molten state from which the solvent used in the polymerization reaction has been removed or reduced in amount. At the time of extrusion, the aforementioned filler or additive may be mixed in advance into the copolymer, if desired, within a range that does not impair the technical effect of the present invention. The process of removing or reducing the solvent, melting the copolymer, and extruding the copolymer from the die hole is not particularly limited, but may be carried out by a variety of methods depending on the process, working environment, properties of the copolymer, and the type of desired molded solid particles.

One or more options selected from the following may be selected and are preferred in step (I):

Option (a): use of an extruder equipped with a die; Option (b): after performing distillation to remove or reduce the solvent using a kettle (pot) or thin-film evaporator equipped with a pressure reducing device, a die is installed under the kettle or at the outlet of the evaporator, and the copolymer is extruded from the hole of the die by liquid delivery means such as a gear pump; and Option (c): liquid delivery means such as a gear pump is provided on an outlet side of the extruder, and a die is provided at the outlet in order to extrude the copolymer from the hole of the die.

In particular, the production method of the present invention is particularly preferably carried out using an extruder equipped with a die (i.e., option (a) above), and a strand-like extrusion product made of the copolymer can be continuously produced, so this method is suitable for a continuous production method with excellent industrial productivity. Furthermore, even if strands are not formed, one or more options selected from the above options (a) to (c) can be selected for step (I). In the production method of the present invention, a single-screw or multi-screw extruder can be used as the extruder, but a multi-screw extruder is preferred, and a twin-screw extruder is particularly preferred. In addition, the scale of the extruder and the ratio of the barrel length to the aperture (L/D) are not particularly limited, but the L/D value of the extruder may be in a range of 20 to 150, or may be in a range of 20 to 100.

In the production method of the present invention, the thermoplastic copolymer is preferably supplied in the form of a copolymer solution dissolved in at least one type of organic solvent (including a form in which the copolymer solution after synthesis is used as is) from a hopper or the like to an extruder equipped with a die using a pump or the like. In this case, any filler and/or additives may be fed to the extruder. These components may be fed from the same hopper together with the copolymer solution described above, or may be fed from a different raw material inlet while adjusting the feed rate by the flow rate of a pump or the like. In particular, by adjusting the feed rate of a pump or the like, the composition of the molded solid particles obtained in a continuous production process can be kept constant.

The copolymer solution fed to the extruder is subjected to an organic solvent distillation operation in the extruder in order to remove or reduce the amount of one type of organic solvent that was the dispersion medium. The distillation operation is continuously carried out in the barrel of the extruder by kneading accompanied by heating or decompression, and preferably the volatile components, such as organic solvents, have been removed from the copolymer to a desired extent by the time the copolymer reaches the die provided at the outlet of the extruder. Here, the conditions for the distillation operation in the barrel unit of the extruder are not particularly limited, but the set temperature of the barrel unit is

preferably set within a range from a temperature 60°C lower than the boiling point of the organic solvents to a temperature no lower than the boiling points of all the organic solvents. When a plurality of organic solvents are present in the copolymer solution, the temperature may be set within a range from a temperature 60°C lower than the boiling point of the organic solvent having the lowest boiling point to a temperature no less than the boiling point of all of the organic solvents. From the viewpoint of efficient removal of the organic solvents, however, the temperature is particularly preferably set within a range from a temperature 30°C lower to a temperature 60°C higher than the boiling point of the organic solvent having the highest boiling point. For example, when isododecane is distilled off from a copolymer solution in which the copolymer is dispersed only in isododecane having a boiling point of 177°C, the set temperature of the barrel unit may be in a range of 117°C to over 177°C, and is preferably set in a range of 147°C to 237°C. In particular, the temperature inside the barrel is preferably set lower than the boiling point of the organic solvent by a temperature difference of at most 60°C. At temperatures below this temperature, sufficient distillation is not achieved, resulting in a large amount of remaining solvent, which increases the viscosity and makes the surface prone to significant tack. As a result, stranding might not be possible, or even if stranding is possible, cutting the strands with a cutting device having a rotary blade and/or a fixed blade might not be possible. Conversely, setting the temperature inside the barrel too high is not only uneconomical, but also may cause the copolymer itself to readily chemically deteriorate or decompose due to overheating, or the melt viscosity may become too high, so that molding the copolymer into strands at the die outlet is impossible.

In addition, from the viewpoint of efficient removal of the organic solvent, distillation is preferably carried out under reduced pressure conditions, and the degree of vacuum may be set to a desired level, but can be set in a range of 0 to 90 kPa, based on atmospheric pressure (about 101 kPa).

The copolymer from which the organic solvent has been distilled off in the barrel (which may optionally contain other fillers or additives) is heated and kneaded in the extruder until reaching the die, and is extruded in the form of strands. In this case, the thermoplastic copolymer preferably has a melt viscosity in the die in a range of 1 to 10000 Pa·s, so the die outlet temperature of the extruder is particularly preferably set to a temperature 5 to 100°C higher than the glass transition point (Tg) of the thermoplastic copolymer. By setting the die outlet temperature within the above range, the copolymer kneaded in the barrel and from which the organic solvent has been distilled off is in a molten state having the aforementioned melt viscosity, and can be molded into strands without causing molding defects or clogging in the die, and can be cut after optionally cooling in a subsequent process. On the other hand, if the die outlet temperature is too low, copolymer that solidifies in the die may clog the die, such that obtaining strand-shaped molded product is impossible or production is difficult. Conversely, if the die outlet temperature is too high, the copolymer itself may be chemically degraded or decomposed due to overheating, or the strand-like molded product from the die may be easily melted and deformed, resulting in molding defects. Note that forming strands is not mandatory, and the die outlet temperature can be set in the same manner even when the resin is extruded from the die without forming strands.

The strand-like extrusion product obtained in step (I) is cooled in an optional water bath or the like to adjust the hardness to a level suitable for cutting, and then cut into pieces having a major axis of less than 10 mm in the cutting step (II). The step of cutting the strand-like extrusion product is preferably carried out by one or more type of cutting device selected from those with a rotary blade and those with a fixed blade. Incidentally, the cutting step is preferably carried out industrially by a cutting device called a pelletizer, and the strand-like extrusion product is cut into formed solid particles, or in other words, pellets having uniform shape and a major axis of less than 10 mm. It should be noted that tablets, short fibers and molded granules having a major axis of less than 10 mm may be formed by using cutting means other than a pelletizer and optional additional granulation/shaping means.

In addition, when no strands are formed in step (I), the material may be cut immediately after being extruded from the die, and in this case, the material may be cut in a molten state without going through a cooling step, or the material may be cut in a semi-solid state during cooling, and then the molded granules may be obtained. For example, a known hot-cut pelletizer is a device that cuts the material immediately after being extruded from a die without going through a cooling process. After cutting, the material can be cooled by any means such as water cooling, air cooling, or belt cooling to obtain solid molded pellets. A known method for cutting the material in a semi-solid state during cooling immediately after extrusion from a die is underwater cutting (also known as underwater pelletizing).

FIG. 1 attached to the present patent application depicts a schematic diagram of a particularly preferred device for producing molded solid particles (pellets) made of the copolymer of the present invention. In other words, the thermoplastic copolymer solution of the present invention (for example, a solution in which a silicone-acrylate copolymer having a carbosiloxane dendrimer structure obtained by a synthetic reaction is dispersed or dissolved in isododecane or the like) is charged into a hopper and transferred to an extruder by a pump. The copolymer solution is temperature-controlled and kneaded under reduced pressure conditions and volatile components such as organic solvents are distilled off in the barrel of the extruder, so the copolymer solution reaches the die of the extruder in a heated and molten state, and is extruded in the form of strands from the die holes where the temperature is controlled (= step (I)). The molten copolymer extruded in the form of strands is then cooled in a cooling tank, which is a water bath, and solidified to a hardness suitable for cutting. Finally, the solidified strand-like copolymer reaches a cutting device, which is a pelletizer, where the copolymer is cut into uniform pellets having a major axis of less than 10 mm, in the form of molded solid particles (=step (II)), which are

then collected.

Step (III) is a step of melting the thermoplastic copolymer of the present invention and extruding from a droplet former to form droplets in a molten state. One known method for granulating a resin in a molten state is a melt granulation device, and although there is no particular limitation, a known steel belt granulator (also known as a pastillator, belt granulator, belt conveyor granulator, or the like) or a known spray cooling device (also known as a prilling tower granulator, spray chilling device, or the like) can be used. For example, the steel belt granulator disclosed in Japanese Unexamined Patent Application S60-212166 can be used, and the spray cooling means or cooling granulation methods disclosed in Japanese Unexamined Patent Application S61-245832 can be used as a spray cooling device. However, a spray freezing device can also be used, depending on the cooling method, but the present invention is not limited to these methods.

The copolymer may be molded/remolded by a spray granulator such as a spray dryer either as is or after dissolving the aforementioned molded solid particles in a solvent or the like.

[Post-processing or remolding operation]

The molded solid particles according to the present invention are produced by the aforementioned method. Depending on process requirements or quality or specification requirements, the coarsely ground molded solid particles of the copolymer may be optionally re-ground (including for the purpose of obtaining molded and ground particles having a smaller particle size) or melted and re-molded into tablets, granules, sheets, or the like. For the purpose of removing the remaining solvent or monomer, the molded solid particles of the copolymer may be subjected again to the same or a different process (a representative example is a process in which the copolymer is again charged into a hopper or the like and melt-kneaded by an extruder or the like), extruded in the form of strands from a temperature-controlled die hole, and pelletized by cutting or other means.

If strands are not formed during the production of the molded solid particles of the present invention, the material may be cut immediately after being extruded from the die, and in this case, the material may be cut in a molten state without passing through a cooling step, or the material may be cut in a semi-solid state during cooling, and then molded granules may be obtained. The obtained molded solid particles of the copolymer may be optionally classified (typically, using a sieving device equipped with a wire mesh or an air classifier (gravity classifier, inertia classifier, centrifugal force classifier)), based on the process requirements, quality requirements, or specification, either as is or after coarse pulverization or fine pulverization. However, these post-treatment and remolding processes are not limited in any way. In the production method of the present invention, other raw materials may be added as desired, and the mixture may be molded into a sheet, granules, tablets, or other form by press molding or the like. The inorganic fine particles may be mixed with powdered silica or the like to form molded solid particles having silica particles or the like on the surface in order to prevent aggregation.

[Use of molded solid particles: cosmetic materials and production method for cosmetic materials]

The molded solid particles of the present invention are molded solid particles made of a copolymer having a silicon-containing organic group in the molecule and having thermoplasticity, and are readily soluble in solvents acceptable for cosmetic materials. Therefore, the particles can be used without any particular limitation in applications in which these copolymers have been conventionally used.

Specifically, the molded solid particles of the present invention can be suitably used as a cosmetic raw material, and as a copolymer, the particles have properties such as water repellency, cleansability, and film forming properties, similar to those of known acrylic silicone dendrimer copolymers. For this reason, for example, some or all of the vinyl-based polymer having a carbosiloxane dendrimer structure in a side chain as described in Patent No. 4009382 (Japanese Unexamined Patent Application 2000-063225) and the like can be substituted in known formulations. In other words, the present applicant teaches that in cosmetic formulations containing a vinyl polymer having a known carbosiloxane dendrimer structure in a side chain, the corresponding cosmetic formulation can be designed or produced by replacing the vinyl polymer with molded solid particles made of the copolymer of the present invention, or a solution obtained by dissolving or dispersing the molded solid particles in a solvent that is acceptable for cosmetic materials.

Similarly, the copolymer of the present invention may be used by substituting some or all of known formulations proposed for commercial products of vinyl-based polymers having a carbosiloxane dendrimer structure in a side chain. For example, commercially available products include FA 4001 CM Silicone Acrylate, FA 4002 ID Silicone Acrylate, and DOWSIL™ FA 4003 DM Silicone Acrylate, manufactured by Dow Toray Co., Ltd., and cosmetic formulations using these products are known in many products, patents, formulation samples provided by DOW SILICONE CORPORATION, and public information on IP.com, and molded solid particles made of the copolymer of the present invention or a solution obtained by dissolving or dispersing the molded solid particles in a solvent acceptable for cosmetic materials may be, and is preferably, used without any restrictions to replace a portion or all of the vinyl polymer having a carbosiloxane dendrimer structure in the side chain.

When a vinyl polymer having a carbosiloxane dendrimer structure in a side chain in a known cosmetic formulation is replaced with a molded solid particle made of the copolymer of the present invention, the corresponding material in the cosmetic formulation can be simply replaced with the molded solid particle made of the copolymer of the present invention using a typical compounding formulation known to those skilled in the art. This method uses a known formulation and does not impair the performance and properties of the cosmetic formulation. The particles are a solid raw material, so handling and workability are improved, and since the particles do not contain a solvent but are readily soluble in solvents that are acceptable for cosmetic materials, the production process is easy. Furthermore, there are practical benefits in that a cosmetic formulation can be provided with excellent freedom of formulation design and usability.

The cosmetic material containing the molded solid particles of the copolymer of the present invention is particularly preferably prepared through a production process including a step of dissolving or dispersing the molded solid particles of the present invention in a solvent acceptable for cosmetic materials, including the aforementioned solvents.

The cosmetic material containing the molded solid particles (A) of the present invention can include various cosmetic raw materials in addition to the aforementioned copolymer. Suitable examples of other cosmetic raw materials include, but are not limited to, cosmetic materials containing at least one raw material selected from the group consisting of (B) water, (C) alcohol, (D) oil agent, (E) powder or colorant, (F) surfactant, (G) oil-soluble gelling agent, (H) organically modified clay mineral, (I) silicone resin, (J) silicone gum, (K) silicone elastomer, (L) organically modified silicone, (M) ultraviolet light protection component, (N) water-soluble polymer, and (O) alkyl -modified silicone resin wax. The molded solid particles of the present invention may be dissolved or dispersed in one or more of (C) alcohols and/or (D) oil agents as solvents acceptable for cosmetic materials, and may be blended in the cosmetic materials in the form of a copolymer composition containing the copolymer and these dispersion media.

Other components ordinarily used in cosmetic materials can be added to the cosmetic material of the present invention within a range that does not hinder the effect of the present invention, and examples of other such components include: organic resins, moisturizing agents, antiseptic agents, antimicrobial agents, perfumes, salts, antioxidants, pH adjusting agents, chelating agents, refreshing agents, anti-inflammatory agents, skin beautifying components (skin lightening agents, cell activating agents, rough skin improving agents, circulation promoters, skin astringents, anti-seborrheic agents, etc.), vitamins, amino acids, nucleic acids, hormones, and inclusion compounds. These specific examples are common with, but not limited to, those examples specifically disclosed in paragraphs [0100] to [0113] and the like of Japanese Unexamined Patent Application 2011-149017.

The cosmetic material of the present invention can contain a natural plant extract component, a seaweed extract component, and an herbal medicine component in accordance with the purpose thereof. Two or more types of these components may be compounded. These specific examples are common with, but not limited to, those examples specifically disclosed in paragraph [0115] and the like of Japanese Unexamined Patent Application 2011-149017.

Depending on the purpose thereof, the cosmetic material of the present invention may contain, for example, a solvent such as light isoparaffin, ether, LPG, N-methylpyrrolidone, alternative chlorofluorocarbons, and the like.

[Type and Formulation of Cosmetic Material]

Furthermore, the type of cosmetic material and a form of a formulation thereof are not particularly limited, and may be: skin cosmetic materials such as skin care products, antiperspirant products, deodorant products, makeup products, ultraviolet light blocking products, and the like; eyelash cosmetic products, hair cleaning agent products, hair dressing products, hair coloring products, hair nourishing products, hair rinsing products, hair conditioner products, hair treatment products, and other head hair cosmetic products; and hair cosmetic products such as bath cosmetic products and the like. Furthermore, the form thereof is not particularly limited, but may be a solution, emulsion, cream, solid, semi-solid, paste, gel, powder, multilayer, mousse, and water-in-oil or oil-in-water emulsified composition (emulsion composition).

Herein, topical agents are applied to the skin, nails, hair, and the like of the human body, and can be used to treat various diseases by blending a pharmaceutical active component, for example. The cosmetic material is also applied to the skin, nails, hair, and the like of the human body, but are used for cosmetic purposes. Even if the application is a "topical agent", use in reality is possible in the same dosage and administration as a cosmetic material. Therefore, in the cosmetic material in the present application, such topical agents are also described as being included as cosmetic materials. Examples thereof include antiperspirants, skin cleaning agents, skin topical agents, hair cleaning agents, hair topical agents, and the like. Examples of applications as a drug of the topical agent include, but are not limited to, hair growing agents, hair tonics, analgesics, disinfectants, anti-inflammatory agents, refreshing agents, and skin anti-aging agents.

Film forming properties, effect on feel on hair

Since the cosmetic material according to the present invention contains the aforementioned copolymer, a film

having excellent water repellency as well as excellent durability and sebum durability can be formed on skin or hair. In particular, the molded solid particles made of the copolymer of the present invention do not contain an organic solvent and are easily soluble in solvents acceptable for cosmetic materials, allowing for a high degree of freedom in formulation design and enabling the formation of a film on the skin or hair that has excellent water resistance and sebum resistance, thus enabling design of cosmetic materials that provide these functional films.

The skin cosmetic materials can be used on any part of the scalp, face (including lips, eyebrows, and cheeks), fingers, nails, and entire body. More specific examples include: skin cleansing agent products such as cleansing gels, cleansing creams, cleansing foams, facial cleansing creams, eye makeup removers, facial cleansing foams, liquid soaps (body soaps), hand soaps, gelatinous soaps, shaving creams, removers, and anti-acne cosmetics; skincare products such as skin creams, scalp treatments, skin milks, milk lotions, emulsions, facial packs, body powder, essences, shaving lotions, and massage lotions; makeup products such as foundations, liquid foundations, oily foundations, makeup bases, white powders, face powders, blushes, lip creams, rouge, lip gloss, eye-creams, mascara, eyebrow pencils, and eyelash cosmetic products; antiperspirants such as deodorants; and UV light blocking products such as sunscreens and sunburn medicinal agents (suntanning agents).

Examples of the hair cosmetic products include: hair detergents such as shampoos and rinse-in-shampoos; hair dressing products such as hair waxes, hair curl holding agents, setting agents, hair creams, hair sprays, and hair liquids; hair coloring products such as hair dyeing agents, hair color sprays, hair color rinses, and hair color sticks; hair tonic products such as hair tonics, hair treatment essences, and hair packs; and hair-rinse or hair conditioning products such as oil rinses, cream rinses, treatment rinses, hair conditioners, and hair treatments. The bath cosmetic products include a foam bath.

[Other applications]

The molded solid particles of the present invention can also be incorporated into applications other than cosmetic materials, such as various external preparations, paints, coating agents, antifoaming agents, deodorants, and the like. In this case, the molded solid particles can ensure advantages in terms of handling, such as transportation, as a solid raw material with low flammability, by taking advantage of the ease of handling and solubility. The molded solid particles are particularly preferably dissolved in a desired solvent and uniformly blended into the final product.

[Use as a film forming agent]

The molded solid particles of the present invention can be used as a film forming agent in applications other than cosmetic materials, taking advantage of the ease of handling and solubility. In other words, the film can be used in any field so long as the application requires a water repellency effect, oil repellency effect, integrity, strength and scratch resistance on a film. In particular, use is possible as an agent for imparting water repellent effects to paint compositions and fibers, and as an agent for treating inorganic and organic materials. Furthermore, the film forming agent of the present invention can be, if necessary, used in an aqueous environment, and therefore is easy to handle.

Specifically, the film forming agent of the present invention can be handled as a solid raw material and is easily soluble in solvents, and therefore has excellent blending stability and does not impair the film forming properties or usability, and can thus be suitably used as a film forming agent for industrial applications in fiber treatment agents or fabric/textile treatment agents.

## EXAMPLES

Hereinafter, the present invention is described in greater detail through examples, but the present invention is not limited thereto.

Each characteristic in the examples and the like was measured by the following methods.

Method for measuring glass transition temperature (Tg) of copolymer

The solvent dispersion solution of each copolymer was soaked into filter paper cut into strips (size: 1 x 5 cm), which were then dried to prepare samples. The test on the sample filter paper was performed using a dynamic viscoelasticity device, Anoton Paar MCR302, under the following scanning conditions: shear mode, temperature rise rate 3°C/min, frequency 1 Hz, and strain 0.1%. First, the filter paper before impregnation was measured to confirm that no particular transition peaks were observed within the measurement range. Then, the impregnated sample was measured to determine tan $\delta$ at each temperature, and the peak top temperature was taken as the glass transition temperature (Tg) of each copolymer.

Method for measuring non-volatile content (NVC) of molded solid particles (pellets) made of copolymer

1 g of each type of molded solid particles (pellets) was placed on an aluminum dish and left to stand in an oven at 150°C for 1 hour, after which the weight was measured and the non-volatile content (NVC) was calculated according to the following formula.

$$\text{NVC (\%)} = (\text{weight before heating} - \text{weight after heating}) \times 100 / \text{weight before heating}$$

Measurement of melt viscosity

Unless otherwise specified, the melt viscosity in the examples was measured using a Shimadzu CFT-EX series under a pressure condition of 2.452 MPa (unit of melt viscosity: Pa·s).

[Pelletizing process]

The pelletizing process and processing device in the examples (common to both the examples and the comparative examples) was configured by connecting a hopper, a liquid delivery line (including a liquid delivery pump), a twin-screw extruder, a strand die, a water bath (cooling tank), and a pelletizer, as depicted in FIG. 1. Here, the L/D ratio (ratio of length to aperture) of the twin-screw extruder was 53, and the die had three circular extrusion holes with a diameter of 5 mm. The water bath was placed between the strand die and the pelletizer equipped with a cutting device (strand cutter), and the strand-like copolymer extruded in a molten state was cooled/solidified in the water bath and then cut by the pelletizer.

[Preparation of thermoplastic vinyl copolymer solutions No. 1 to No. 3 having a carbosiloxane dendrimer structure in the molecule]

As shown in Table 1, the following copolymer solutions No. 1 to No. 3 were used in the Examples and Comparative Examples. The copolymer concentration (=non-volatile content) in each solution was standardized to 40 mass%.

Copolymer solution No. 1: DOWSIL(TM) FA 4002 ID (manufactured by Dow Toray Co., Ltd., commercially available product: 40 mass% diluted with isododecane (ID))
Copolymer solution No. 2: DOWSIL(TM) FA 4004 ID (Dow Toray Co., Ltd., commercially available product: 40% by weight diluted with isododecane (ID))
Copolymer solution No. 3: 40 mass% dilution of the copolymer obtained in
Synthesis Example 1 below with isopropanol (IPA)
Synthesis Example 1: Copolymer Solution No. 3

A 50-liter flask equipped with a stirrer, a thermometer, and a reflux condenser was charged with 11.62 kg of isopropyl alcohol (IPA), thoroughly degassed by bubbling with nitrogen gas, and heated to 80°C. A mixture containing 7.7 kg of methyl methacrylate, 0.7 kg of n-butyl acrylate, and 5.6 kg of a monomer having a carbosiloxane dendrimer structure expressed by the following formula

[Chem. Fig. 30]

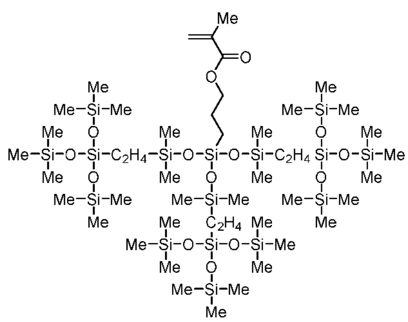

and a mixture of 1.12 kg of 2,2'-azobis-2-methylbutyronitrile (Fuji Film Wako Co., Ltd.) and 7.42 kg of IPA were added at a constant rate over a period of 2 hours, and finally the feed line was rinsed with 1.96 kg of IPA. After the entire amount was added, the mixture was heated and stirred for 6 hours under a nitrogen atmosphere. The reaction product was analyzed by liquid chromatography, and the conversion rate of polymerization was found to be 96%. The obtained copolymer solution No. 3 was a pale yellow transparent liquid with a viscosity of 116 mPa·s as measured using a rotational viscometer at room temperature and a copolymer concentration (= non-volatile content) of 40.0 mass%.

[Comparative Example 1]

During the pelletizing process of the example, 20 kg of copolymer solution No. 2 (8 kg based on non-volatile solid content) was continuously fed into a twin-screw extruder at a feed rate of 5 kg/hr (= extrusion rate of 2 kg/hr), the maximum temperature in the barrel was set to 200°C, the barrel temperature in the distillation unit near the die was set to 100°C, and the degree of vacuum in the distillation unit was set to 70 kPa. The extruder speed was fixed at 101 rpm. After removing the solvent and volatile matter in the extruder (barrel), the molten thermoplastic copolymer was extruded from the die and passed through a water bath at 25°C. However, the extruded strands were very soft and thin and quickly broke, so obtaining molded solid particles with a major axis of less than 10 mm was impossible. The non-volatile content (NVC) of these defective moldings was found to be 88.1 mass%, indicating that the organic solvents had not been sufficiently removed. Furthermore, the Tg (measured value) of the copolymer in Copolymer Solution No. 2 was relatively low at 40.5°C, and when the copolymer contained a large amount of organic solvent as described above, the copolymer was found to be too soft to be molded/cut into strands, which was also a cause of molding defects.

[Comparative Example 2]

During the pelletizing process of the example, 20 kg of copolymer solution No. 1 (8 kg based on non-volatile solid content) was continuously fed into a twin-screw extruder at a feed rate of 5 kg/hr (= extrusion rate of 2 kg/hr), the maximum temperature in the barrel was set to 200°C, the barrel temperature in the distillation unit near the die was set to 100°C, and the degree of vacuum in the distillation unit was set to 70 kPa. **The** extruder speed was fixed at 101 rpm. After removing the solvent and volatile matter in the extruder (barrel), the molten thermoplastic copolymer was extruded from the die and solidified by passing through a water bath at a water temperature of 25°C. An attempt was made to cut the solidified strands using a pelletizer, but some of the strands adhered to and wrapped around the rollers that fed the strands due to tack on the strand surface. Furthermore, the strands themselves were soft and could not be cut in the pelletizer, so molded solid particles having a major axis of less than 10 mm could not be obtained. **The** non-volatile content (NVC) of these defective moldings was measured and found to be 90 mass%, indicating that the organic solvents had not been completely removed, which was the cause of the molding defects.

[Example 1]

During the pelletizing process of the example, 20 kg of copolymer solution No. 1 (8 kg based on non-volatile solid content) was continuously fed into a twin-screw extruder at a feed rate of 5 kg/hr (= extrusion rate of 2 kg/hr), the maximum temperature in the barrel was set to 200°C, the barrel temperature in the distillation unit near the die was set to 150°C, and the degree of vacuum in the distillation unit was set to 70 kPa. The extruder speed was fixed at 101 rpm. After removing the solvent and volatile matter in the extruder (barrel), the molten thermoplastic copolymer was extruded from the die and solidified by passing through a water bath at a water temperature of 25°C. The solidified strands could be easily cut with a pelletizer and molded into uniform shapes, yielding molded solid particles (pellets) with a diameter of 4 mm and a length of 3 to 5 mm. The difference between Example 1 and Comparative Example 2 was that the organic solvent was sufficiently removed from the copolymer in Example 1 by increasing the temperature inside the barrel, and the non-volatile content (NVC) of the obtained molded solid particles was 99.4 mass% (see Table 1).

[Example 2]

The procedure of Example 1 was repeated except that Copolymer Solution No. 1 was replaced with the same amount of Copolymer Solution No. 2, and solid particles (pellets) of uniform shape having a diameter of 4 mm and a length of 3 to 5 mm were obtained. The non-volatile content (NVC) of the obtained molded solid particles was 97.7 mass% (see Table 1).

[Example 3]

The procedure of Example 1 was repeated except that Copolymer Solution No. 1 was replaced with the same

amount of Copolymer Solution No. 3, and the temperature of the distillation barrel near the die was set to 100°C. Molded solid particles (pellets) having a uniform shape and a diameter of 4 mm and a length of 3 to 5 mm were obtained. The non-volatile content (NVC) of the obtained molded solid particles was 99.0 mass% (see Table 1).

The process conditions and results for Examples 1 to 3 and Comparative Examples 1 and 2 are summarized in Table 1 below. In the pelletizing process of the examples, the organic solvent must be thoroughly removed from the copolymer in order to avoid molding defects. From the processing perspective, the barrel unit, where the solvent is distilled off, may be set to a high temperature, or an approach can be taken in which a low-boiling point solvent (e.g. IPA) that is easy to distill off is used, and the barrel unit is set to a temperature that matches the boiling point of the solvent.

[Table 1]

| | Examples | Example 1 | Example 2 | Comparative example 1 | Comparative example 2 | Example 3 |
|---|---|---|---|---|---|---|
| Copolymer | Copolymer solution No. | No. 1 | No.2 | No.2 | No. 1 | No.3 |
| | Tg | 87 | 40.5 | 40.5 | 87 | 66.5 |
| | Melt viscosity at die outlet temperature (Pa·s) | 330 | 5.5 | 722 | 7000 | 793 |
| Diluted solvent | Types of diluents | IDD | IDD | IDD | IDD | IPA |
| | Boiling point of diluent [°C] | 182 | 182 | 182 | 182 | 82 |
| Temperature setting in the extruder | Temperature of distillation unit barrel (Tvac) | 150 | 150 | 100 | 100 | 100 |
| | Die outlet temperature (Tout) | 150 | 150 | 100 | 104 | 100 |
| | Diluent boiling point - Tvac | 32 | 32 | 82 | 82 | -18 |
| | Boiling point of diluent - Tg | 95 | 141.5 | 141.5 | 95 | 15.5 |
| Items related to pelletizing | NVC (mass%) | 99.4 | 97.7 | 88.1 | 90 | 99 |
| | Stranding | Good | Good | Not Applicable | Good | Good |
| | Presence or absence of tack during pelletizing (rolling) | Good (no tack) | Good (no tack) | - | Slightly inferior (with tack) | Good (no tack) |
| | Pelletizing (can be cut) | Good | Good | - | Not Applicable | Good |

[Examples 4-1 to 4-3: Evaluation of solubility of pellets containing copolymer]

The pellets made of each of the copolymers obtained in Examples 1 to 3 were evaluated for solubility in solvents acceptable for cosmetic use (including oil agents, organic solvents, and liquid ultraviolet light protection components). Specifically, 0.5 g of each pellet and 2 g of a prescribed solvent (oil, organic solvent) were placed in a 20 cc vial, and the vial was left to stand in an oven heated to 50°C for 30 minutes to confirm whether the pellets had dissolved uniformly.

The results showed that the pellets of the present example dissolved uniformly in all of the following solvents simply by being left to stand under the above conditions, thus demonstrating easy solubility in a variety of solvents.

<u>Solvents evaluated:</u>

Ethanol
Decamethylcyclopentasiloxane (DOWSIL(TM) SH 245 Fluid),
Dimethicone (DOWSIL(TM) SH 200C Fluid 2cs),
Caprylyl Methicone (DOWSIL(TM) FZ-3196 Fluid),
Phenyl trimethicone (DOWSIL(TM) SH 556 Fluid),
Isododecane (PUROLAN IDD, LANXESS Distribution GmbH),
Undecane and Tridecane (Cetiol(R) Ultimate, BASF Japan),
CAPRYLIC/CAPRIC TRIGLYCERIDE (FineNeo-MCT, Nippon Fine Chemical), ETHYLHEXYL METHOXYCINNAMATE (Uvinul(R) MC 80, BASF),
Butyl acetate (Fujifilm Wakosha)

The above results show that the molded solid particles (for example, pellets) of the thermoplastic copolymer having a silicon-containing organic group in a molecule according to the present invention are easily soluble in a variety of liquid solvents used in cosmetic materials, and therefore have excellent handling properties, freedom in formulation design, and blending stability. In addition, the molded solid particles are organic solvent-free, so there are few concerns about fire and safety issues related to transportation and storage, and after blending, there is no deterioration in the feel of use or degradation of feel due to organic solvents. Furthermore, cosmetic raw materials can be blended by being dispersed or dissolved in an oil agent different from the diluent used in commercially available acrylic silicone products, and the same oil agent can be blended at a higher concentration than in commercially available products. For example, there is strong expectation that the design of cosmetic formulations and the feel of use that were not achievable with conventional products can be achieved.

**DESCRIPTION OF CODES**

1. Hopper
2. Plunge pump
3. Twin-screw extruder
4. Strand die
5. Copolymer (material) extruded in strand form
6. Cooling tank (water cooling type)
7. Cutting device for pelletizing (pelletizer device)
8. Molded solid particles of copolymer molded into pellet form

Note: "Vac. and condense" in the figure refers to the distillation unit in the barrel of the twin-screw extruder where the solvent in the copolymer solution is removed and the solid content is concentrated.

## Claims

**1.** Molded solid particles, comprising a thermoplastic copolymer made by copolymerizing:

(a1) an unsaturated monomer having one radical polymerizable organic group and one silicon-containing organic group in the molecule; and
(a2) an unsaturated monomer having one radical polymerizable vinyl group in the molecule, which is different from component (a1);
wherein the major axis of the particles is less than 10 mm.

**2.** The molded solid particles according to claim 1, in one or more forms selected from the group consisting of pellets, tablets, short fibers, and molded granules.

**3.** The molded solid particles according to claim 1, wherein the thermoplastic copolymer has a glass transition point (Tg) in a range of 30 to 120°C.

**4.** The molded solid particles according to claim 1, wherein the thermoplastic copolymer has, in a molecule, a silicon-containing organic group having at least one structure selected from a carbosiloxane dendrimer structure and a siloxane macromonomer structure.

5. The molded solid particles according to claim 1, wherein at least a portion of component (a1) is an unsaturated monomer having a carbosiloxane dendrimer structure expressed by the following formula:

[Chem. Fig. 1]

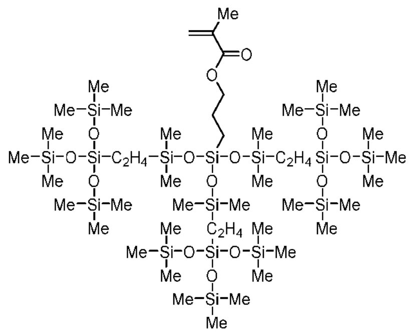

(wherein Me is a methyl group).

6. Use of the molded solid particles according to any one of claims 1 to 5 as a cosmetic raw material.

7. Use of the molded solid particles according to any one of claims 1 to 5 as a film forming agent.

8. A cosmetic material, comprising the molded solid particles according to any one of claims 1 to 5.

9. A method for producing a cosmetic material, comprising a step of dissolving or dispersing the molded solid particles according to any one of claims 1 to 5 in a solvent acceptable for cosmetic materials.

10. A method for producing the molded solid particles according to any one of claims 1 to 5, wherein the method includes a step of extruding while molding the thermoplastic copolymer.

11. The method for producing the molded solid particles according to claim 10, wherein the outlet temperature during extrusion is set so that the melt viscosity of the thermoplastic copolymer is within a range of 0.1 to 10,000 Pa·s.

12. The method for producing the molded solid particles according to claim 10, which is a continuous production method.

13. The method for producing the molded solid particles according to claim 10, comprising:

a step (I) of extruding a thermoplastic copolymer from a die in a form of strands; and
a step (II) of cutting the strand-like extrusion product obtained in step (I).

14. The method for producing the molded solid particles according to claim 13, wherein step (I) of extruding the thermoplastic copolymer from a die in the form of strands is performed using a single-screw or multi-screw extruder equipped with a die.

15. The method for producing the molded solid particles according to claim 14, wherein in the step (I) of extruding a thermoplastic copolymer from a die in the form of strands using a single-screw or multi-screw extruder equipped with a die, the thermoplastic copolymer is in the form of a copolymer solution dissolved in at least one type of organic solvent, and the temperature setting of the extruder satisfies both of the following conditions:

i) the die outlet temperature of the extruder is set to a temperature 5 to 100°C higher than the glass transition point (Tg) of the thermoplastic copolymer; and
ii) in order to distill off the organic solvent from the copolymer solution, the set temperature of the barrel in the extruder is set within a range from a temperature 60°C lower than the boiling point of the organic solvent (the lowest boiling point if a plurality of organic solvents are present) to a temperature higher than the boiling points of all the organic solvents.

16. The method for producing the molded solid particles according to claim 13, wherein step (II) of cutting the strand-like extrusion product obtained in step (I) is performed by using one or more types of cutting devices selected from rotary blades and fixed blades.

17. The method for producing the molded solid particles according to claim 10, further comprising:

a step (III) of extruding a thermoplastic copolymer from a droplet former; and
a step (IV) of cooling and solidifying the extrusion product of step (III).

18. The method for producing the molded solid particles according to claim 17, wherein step (III) of extruding the thermoplastic copolymer from the droplet former and the step (IV) of cooling and solidifying the extrusion product in step (III) are performed by a melt granulation device.

[FIG. 1]

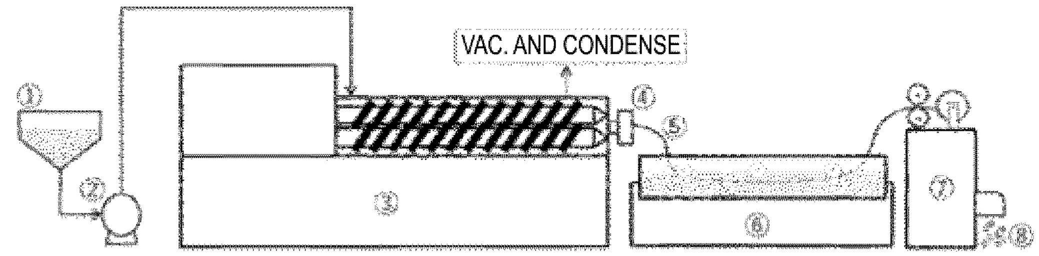

**INTERNATIONAL SEARCH REPORT**

International application No. **PCT/JP2023/041954**

**A. CLASSIFICATION OF SUBJECT MATTER**

***C08J 3/12***(2006.01)i
FI: C08J3/12 Z

According to International Patent Classification (IPC) or to both national classification and IPC

**B. FIELDS SEARCHED**

Minimum documentation searched (classification system followed by classification symbols)

C08J3/12

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y<br><br>A | WO 2022/138330 A1 (DOW TORAY CO., LTD.) 30 June 2022 (2022-06-30)<br>claims, paragraphs [0014]-[0015], [0041]-[0046], [0066], [0107], examples | 1-14, 16-18<br><br>15 |
| Y<br><br>A | JP 2016-028053 A (DOW CORNING CORPORATION) 25 February 2016 (2016-02-25)<br>claims, paragraphs [0032]-[0033], [0072], examples | 1-14, 16-18<br><br>15 |
| A | JP 2003-226611 A (DOW CORNING TORAY SILICONE CO LTD) 12 August 2003 (2003-08-12)<br>entire text | 1-18 |

☐ Further documents are listed in the continuation of Box C.    ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 January 2024** | **30 January 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**
**Information on patent family members**

International application No.
**PCT/JP2023/041954**

| Patent document cited in search report | Publication date (day/month/year) | Patent family member(s) | Publication date (day/month/year) |
|---|---|---|---|
| WO 2022/138330 A1 | 30 June 2022 | CN 116529275 A | |
| JP 2016-028053 A | 25 February 2016 | US 2012/0171264 A1<br>claims, paragraphs [0033]-[0034], [0073], examples | |
| JP 2003-226611 A | 12 August 2003 | US 2005/0008597 A1<br>entire text | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000063225 A
- JP 2018518505 A
- JP 2009149880 A
- JP S60212166 A
- JP S61245832 A
- JP 4009382 B
- JP 2011149017 A